(19) Europäisches Patentamt
European Patent Office
Office européen des brevets

(11) **EP 3 650 848 A1**

(12) **EUROPEAN PATENT APPLICATION**

(43) Date of publication:
**13.05.2020 Bulletin 2020/20**

(51) Int Cl.:
***G01N 27/417*** *(2006.01)*

(21) Application number: **19207122.3**

(22) Date of filing: **05.11.2019**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **07.11.2018 JP 2018210004**

(71) Applicant: **TOYOTA JIDOSHA KABUSHIKI KAISHA**
**Toyota-shi, Aichi-ken, 471-8571 (JP)**

(72) Inventors:
• **AOKI, Keiichiro**
  **Aichi-ken 471-8571 (JP)**
• **TAKAOKA, Kazuya**
  **Aichi-ken 471-8571 (JP)**

(74) Representative: **D Young & Co LLP**
**120 Holborn**
**London EC1N 2DY (GB)**

(54) **FAILURE DETECTION APPARATUS FOR GAS SENSOR AND FAILURE DETECTION METHOD FOR GAS SENSOR**

(57) A failure detection apparatus for a gas sensor includes an electronic control unit (12) configured to: perform control so that a predetermined voltage, which is a positive voltage and is a voltage lower than a limiting current range when the gas sensor (10) does not fail, is applied between a pair of electrodes (102, 103); acquire a determination current, which is a current flowing between the pair of electrodes when the predetermined voltage is applied between the pair of electrodes; and determine whether the gas sensor fails based on the acquired determination current.

FIG. 9

**Description**

BACKGROUND OF THE INVENTION

1. Field of the Invention

The invention relates to a failure detection apparatus for a gas sensor and a failure detection method for a gas sensor.

2. Description of Related Art

In recent years, a limiting current gas sensor configured to detect the concentration of oxygen or oxides (for example, water vapor, a nitrogen oxide, or a sulfur oxide) contained in detection target gas is available. The limiting current gas sensor includes a sensor element including a solid electrolyte having oxide ion conductivity, a pair of electrodes attached to the solid electrolyte, and a diffusion control portion configured to control diffusion of the detection target gas to guide the detection target gas to one of the electrodes. The limiting current gas sensor may be used as an oxygen concentration sensor configured to detect the concentration of oxygen contained in exhaust gas from an internal combustion engine.

Japanese Unexamined Patent Application Publication No. 2004-019542 (JP 2004-019542 A) discloses the following technology as a technology for detecting failure in the oxygen concentration sensor arranged downstream of an exhaust gas catalyst in an exhaust passage of the internal combustion engine. An air-fuel ratio sensor is arranged upstream of the exhaust gas catalyst. The oxygen storage capacity of the exhaust gas catalyst is estimated by summing up the amount of oxygen flowing into the exhaust gas catalyst during a period in which a detection signal from the oxygen concentration sensor indicates a rich oxygen concentration and a detection signal from the air-fuel ratio sensor indicates a lean air-fuel ratio. Determination is made that the oxygen concentration sensor fails when the estimated oxygen storage capacity exceeds a predetermined threshold.

SUMMARY OF THE INVENTION

The related art described above has a constraint that the failure in the oxygen concentration sensor cannot be detected unless the concentration of a predetermined component contained in the detection target gas is a specific concentration.

The invention provides a technology in which failure in a limiting current gas sensor configured to detect the concentration of a predetermined component contained in detection target gas can be detected irrespective of the concentration of the predetermined component contained in the detection target gas in a failure detection apparatus for the gas sensor.

The invention provides a technology in which failure in a limiting current gas sensor is detected focusing on the fact that, when a predetermined voltage that is a voltage lower than a limiting current range and is a positive voltage is applied between a pair of electrodes of the gas sensor, a current flowing between the electrodes exhibits a remarkable difference between a case where the gas sensor fails and a case where the gas sensor does not fail.

A first aspect of the invention relates to a failure detection apparatus for a gas sensor. The gas sensor includes a sensor element including a solid electrolyte having oxide ion conductivity, a pair of electrodes attached to the solid electrolyte, and a diffusion control portion that control a rate of diffusion of detection target gas to guide the detection target gas to one of the pair of electrodes. The gas sensor is a limiting current gas sensor configured to detect a concentration of a predetermined component contained in the detection target gas. The failure detection apparatus includes an electronic control unit. The electronic control unit is configured to perform control so that a predetermined voltage is applied between the pair of electrodes. The predetermined voltage is a positive voltage and is a voltage lower than a limiting current range when the gas sensor does not fail. The electronic control unit is configured to acquire a determination current that is a current flowing between the pair of electrodes when the predetermined voltage is applied between the pair of electrodes. The electronic control unit is configured to determine whether the gas sensor fails based on the acquired determination current.

The "limiting current range" is a range of the voltage applied between the pair of electrodes (may hereinafter be referred to as "applied voltage"), in which a limiting current characteristic emerges between the applied voltage and the current flowing between the electrodes (may hereinafter be referred to as "electrode current"). The operation to "determine whether the gas sensor fails based on the determination current" is not limited to an operation of detecting the failure in the gas sensor by comparing the determination current with a determination threshold, but may be an operation of detecting the failure in the gas sensor by calculating a resistance value between the pair of electrodes from the determination current and comparing the resistance value with a determination threshold.

When the voltage in the limiting current range is applied between the pair of electrodes in a state in which the limiting current gas sensor does not fail, a current (limiting current) having a magnitude proportional to the concentration of the predetermined component contained in the detection target gas flows between the electrodes. The electrode current obtained when the voltage in the limiting current range is applied between the pair of electrodes is unlikely to exhibit a remarkable difference between the case where the gas sensor fails and the case where the gas sensor does not fail. The electrode current (determination current) obtained when the voltage (predetermined voltage) that is lower than the limiting current range and is the positive voltage is applied between the pair of electrodes is likely to exhibit a remarkable difference between the case where the gas sensor fails and the case where the gas sensor does not fail. This difference occurs irrespective of the concentration of the predetermined component contained in the detection target gas. According to the failure detection apparatus of the aspect described above, the failure in the gas sensor can be detected irrespective of the concentration of the predetermined component contained in the detection target gas by performing failure detection for the gas sensor based on the determination current.

In the aspect described above, the gas sensor may be arranged in an exhaust passage of an internal combustion engine, and may be configured to detect a concentration of oxygen serving as the predetermined component, which is contained in exhaust gas serving as the detection target gas. The electronic control unit may be configured to determine whether the gas sensor fails based on a failure characteristic. The failure characteristic is a characteristic in which the determination current is larger in a case where the gas sensor fails than a case where the gas sensor does not fail. Thus, the failure in which the failure characteristic emerges can be detected.

For example, the failure characteristic is likely to emerge in the event of failure in which the exhaust gas serving as the detection target gas comes into contact with not only one electrode out of the pair of electrodes but also the other electrode. In the aspect described above, one electrode out of the pair of electrodes may be arranged so as to face a reference gas chamber into which air is introduced. The other electrode out of the pair of electrodes may be arranged so as to be exposed to the exhaust gas that serves as the detection target gas and is introduced via the diffusion control portion. The electronic control unit may be configured such that a voltage at which the failure characteristic emerges in an event of failure is applied between the pair of electrodes as the predetermined voltage. The failure is failure due to entry of the exhaust gas into the reference gas chamber. Thus, the failure due to the entry of the exhaust gas into the reference gas chamber can be detected more securely.

In the aspect described above, the predetermined voltage may be a voltage at which, when an air-fuel ratio of the exhaust gas is a lean air-fuel ratio a positive or negative sign of the determination current obtained when the exhaust gas does not enter the reference gas chamber is opposite to a positive or negative sign of the determination current obtained when the exhaust gas enters the reference gas chamber. The lean air-fuel ratio may be leaner than a stoichiometric air-fuel ratio. In the aspect described above, the predetermined voltage may be 0.1 volts. According to the aspect described above, the failure detection accuracy can be increased because the failure characteristic emerges more securely in the event of the failure due to the entry of the exhaust gas into the reference gas chamber.

In the aspect described above, the electronic control unit may be configured to determine that the gas sensor fails when the acquired determination current is larger than a predetermined threshold. In the aspect described above, the electronic control unit may be configured to calculate a resistance value between the pair of electrodes based on the acquired determination current and the predetermined voltage. The electronic control unit may be configured to determine that the gas sensor fails when the calculated resistance value is larger than a predetermined upper limit value or when the calculated resistance value is smaller than a predetermined lower limit value.

In the aspect described above, the gas sensor may further include a heater configured to heat the sensor element. The electronic control unit may be configured to control the heater so as to increase a temperature of the sensor element to a temperature equal to or higher than a predetermined temperature, which is higher than an activation temperature of the sensor element, when the predetermined voltage is applied between the pair of electrodes. The "activation temperature of the sensor element" is a temperature at which the solid electrolyte exhibits oxygen ion conductivity.

In the state in which the temperature of the sensor element is a temperature equal to or higher than the activation temperature, the determination current obtained when the exhaust gas does not enter the reference gas chamber is even smaller and the determination current obtained when the exhaust gas enters the reference gas chamber is even larger in a case where the temperature of the sensor element is high when the predetermined voltage is applied between the pair of electrodes than a case where the temperature of the sensor element is low when the predetermined voltage is applied between the pair of electrodes. That is, in the state in which the temperature of the sensor element is the temperature equal to or higher than the activation temperature, the difference in the determination current between the case where the exhaust gas does not enter the reference gas chamber and the case where the exhaust gas enters the reference gas chamber is even larger in the case where the temperature of the sensor element is high when the predetermined voltage is applied between the pair of electrodes than the case where the temperature of the sensor element is low when the predetermined voltage is applied between the pair of electrodes. Thus, the failure detection accuracy can be increased if the heater is controlled so that the temperature of the sensor element is equal to or higher than the predetermined temperature when the predetermined voltage is applied between the pair of electrodes.

For example, the predetermined temperature may be set within a range from 750°C to 850°C. The sensor element of the gas sensor is active at about 600°C. When the temperature of the sensor element is increased to 750°C to 850°C higher than 600°C, the difference in the determination current between the case where the exhaust gas does not enter the reference gas chamber and the case where the exhaust gas enters the reference gas chamber increases more remarkably because the resistance value between the pair of electrodes decreases greatly. As a result, the failure detection accuracy can be increased more securely. When the heater heats the sensor element, the heater is controlled so that the temperature of the sensor element does not exceed 900°C. This is because the measurement accuracy of the gas sensor decreases or the gas sensor thermally deteriorates when the temperature of the sensor element exceeds 900°C.

A second aspect of the invention relates to a failure detection method for a gas sensor. The gas sensor includes a sensor element including a solid electrolyte having oxide ion conductivity, a pair of electrodes attached to the solid electrolyte, and a diffusion control portion that controls a rate of diffusion of detection target gas to guide the detection target gas to one of the pair of electrodes. The gas sensor is a limiting current gas sensor configured to detect a concentration of a predetermined component contained in the detection target gas. The failure detection method includes: performing, by an electronic control unit, control so that a predetermined voltage is applied between the pair of electrodes; acquiring, by the electronic control unit, a determination current that is a current flowing between the pair of electrodes when the predetermined voltage is applied between the pair of electrodes; and determining, by the electronic control unit, whether the gas sensor fails based on the determination current. The predetermined voltage is a positive voltage and is a voltage lower than a limiting current range when the gas sensor does not fail.

According to the invention, the failure in the limiting current gas sensor can be detected irrespective of the concentration of the predetermined component contained in the detection target gas.

BRIEF DESCRIPTION OF THE DRAWINGS

Features, advantages, and technical and industrial significance of exemplary embodiments of the invention will be described below with reference to the accompanying drawings, in which like numerals denote like elements, and wherein:

FIG. 1 is a schematic diagram illustrating structures of an internal combustion engine and its intake and exhaust system to which a gas sensor that is a target of the invention is applied;
FIG. 2 is a schematic diagram illustrating a structure of an A/F sensor;
FIG. 3 is a diagram illustrating correlations between an applied voltage Vev and an electrode current Iec;
FIG. 4 is a diagram illustrating a correlation between a limiting current Ilc and an air-fuel ratio A/F;
FIG. 5 is a diagram illustrating an example of a state in which a sensor element is cracked;
FIG. 6 is a diagram illustrating correlations between the applied voltage Vev and the electrode current Iec when exhaust gas enters a reference gas chamber in a state in which an air-fuel ratio of the exhaust gas is a lean air-fuel ratio;
FIG. 7 is a diagram illustrating correlations between the applied voltage Vev and the electrode current Iec when the exhaust gas enters the reference gas chamber in a state in which the air-fuel ratio of the exhaust gas is a stoichiometric air-fuel ratio;
FIG. 8 is a diagram illustrating correlations between the applied voltage Vev and the electrode current Iec when the exhaust gas enters the reference gas chamber in a state in which the air-fuel ratio of the exhaust gas is a rich air-fuel ratio;
FIG. 9 is a flowchart illustrating a processing routine to be executed when an ECU performs failure detection processing in a first embodiment;
FIG. 10 is a diagram illustrating correlations between the applied voltage Vev and the electrode current Iec when the exhaust gas does not enter the reference gas chamber;
FIG. 11 is a diagram illustrating correlations between the applied voltage Vev and the electrode current Iec when the exhaust gas enters the reference gas chamber; and
FIG. 12 is a flowchart illustrating a processing routine to be executed when the ECU performs failure detection processing in a second embodiment.

DETAILED DESCRIPTION OF EMBODIMENTS

Specific embodiments of the invention are described below with reference to the drawings. Dimensions, materials, shapes, relative arrangement, and the like of components described in the embodiments are not intended to limit the technical scope of the invention unless otherwise noted.

First Embodiment

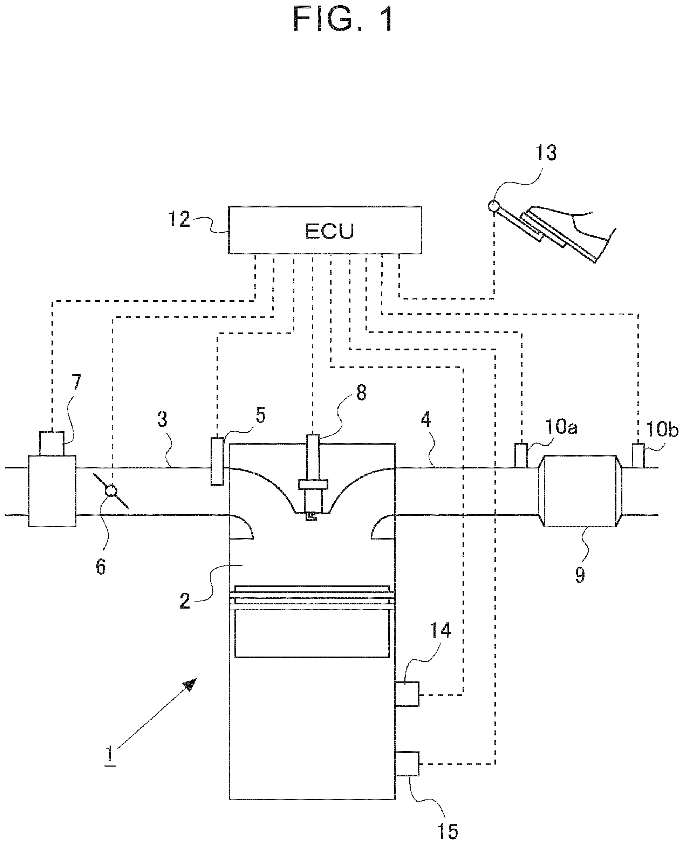

A first embodiment of the invention is described with reference to FIG. 1 to FIG. 9. Description is given of an example in which the invention is applied to an air-fuel ratio (A/F) sensor configured such that detection target gas is exhaust gas from an internal combustion engine mounted on a vehicle and the concentration of oxygen contained in the exhaust gas (A/F) is detected as the concentration of a predetermined component.

FIG. 1 is a schematic diagram illustrating structures of the internal combustion engine and its intake and exhaust system to which a gas sensor that is a target of the invention is applied. An internal combustion engine 1 illustrated in FIG. 1 is a spark ignition type internal combustion engine (gasoline engine) including a plurality of cylinders 2. The internal combustion engine 1 may be a compression ignition type internal combustion engine (diesel engine) using light oil as fuel.

An intake passage 3 and an exhaust passage 4 leading into the cylinder 2 are connected to the internal combustion engine 1. A fuel injection valve 5 is attached to a part of the intake passage 3 near the internal combustion engine 1 (for example, an intake port or an intake manifold). The fuel injection valve 5 may be arranged at a position where fuel can directly be injected into the cylinder 2. The fuel injected from the fuel injection valve 5 is mixed with air flowing through the intake passage 3 to form an air-fuel mixture. The air-fuel mixture is combusted by being ignited by a spark plug 8 in the cylinder 2.

A throttle valve 6 is provided upstream of the fuel injection valve 5 in the intake passage 3. The throttle valve 6 adjusts the intake amount of the internal combustion engine 1 by changing the passage sectional area of the intake passage 3. An airflow meter 7 is provided upstream of the throttle valve 6 in the intake passage 3. The airflow meter 7 measures the mass of fresh air (air) flowing through the intake passage 3.

A catalyst casing 9 that houses an exhaust gas catalyst is arranged midway along the exhaust passage 4. Examples of the exhaust gas catalyst housed in the catalyst casing 9 include a three-way catalyst, a $NO_X$ storage reduction (NSR) catalyst, a selective catalytic reduction (SCR) catalyst, and an oxidation catalyst. A first A/F sensor 10a is arranged upstream of the catalyst casing 9 in the exhaust passage 4. A second A/F sensor 10b is arranged downstream of the catalyst casing 9 in the exhaust passage 4. As described above, the first A/F sensor 10a and the second A/F sensor 10b (may hereinafter be referred to collectively as "A/F sensors 10") detect the concentration of oxygen contained in the exhaust gas, and can be regarded as the gas sensor according to the invention. The specific structure of the A/F sensor 10 is described later.

An electronic control unit (ECU) 12 is provided together with the internal combustion engine 1 constructed as described above. The ECU 12 is constituted by a central processing unit (CPU), a read-only memory (ROM), a random-access memory (RAM), a backup RAM, and the like. The ECU 12 is electrically connected not only to the airflow meter 7 and the A/F sensors 10 but also to various other sensors such as an accelerator position sensor 13, a crank position sensor 15, and a coolant temperature sensor 14. The accelerator position sensor 13 measures an operation amount of an accelerator pedal (accelerator operation amount). The crank position sensor 15 measures a rotational position of an output shaft (crankshaft) of the internal combustion engine 1. The coolant temperature sensor 14 measures a temperature of a coolant circulating through the internal combustion engine 1. Measured values of the various sensors are input to the ECU 12.

The ECU 12 is electrically connected to various devices mounted on the internal combustion engine 1 (for example, the fuel injection valve 5, the throttle valve 6, and the spark plug 8), and can control those devices based on the measured values of the various sensors. For example, the ECU 12 calculates a target fuel injection amount, a target fuel injection timing, a target ignition timing, and a target throttle opening degree based on an engine speed calculated from the measured value of the crank position sensor 15, the accelerator operation amount measured by the accelerator position sensor 13, and the coolant temperature measured by the coolant temperature sensor 14, and controls the various devices based on the target values.

Structure of A/F Sensor

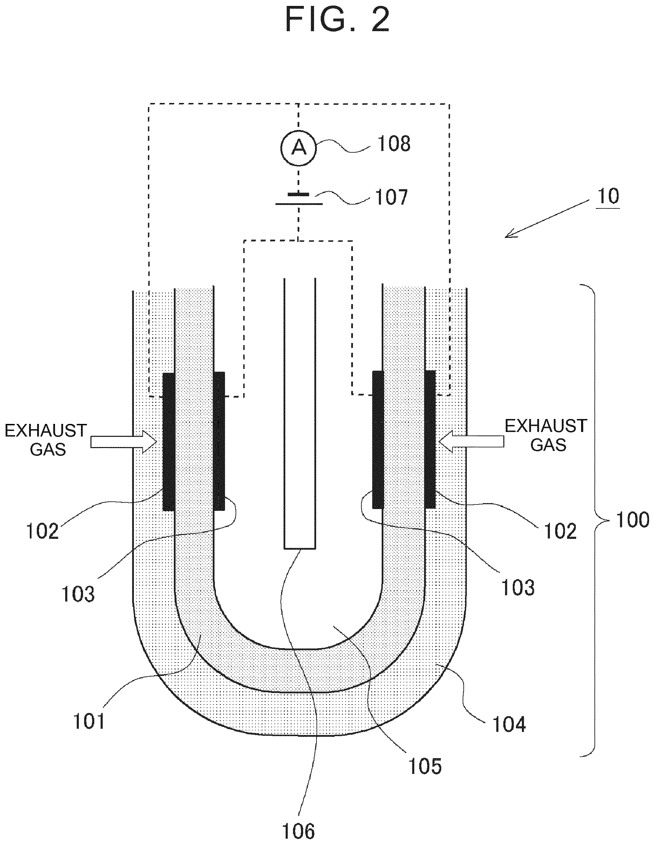

Next, the structure of the A/F sensor 10 is described with reference to FIG. 2. FIG. 2 is a schematic diagram illustrating a structure of the A/F sensor 10 of this embodiment. As illustrated in FIG. 2, the A/F sensor 10 of this embodiment includes a sensor element 100 including a bottomed-cylindrical solid electrolyte layer 101, an annular exhaust gas-side electrode 102, an annular air-side electrode 103, a diffusion control layer 104, a reference gas chamber 105, and a heater 106. The exhaust gas-side electrode 102 is attached to the outer peripheral surface of the solid electrolyte layer 101. The air-side electrode 103 is attached to the inner peripheral surface of the solid electrolyte layer 101 at a part that faces the exhaust gas-side electrode 102. The diffusion control layer 104 is formed so as to cover the outer wall surface of the solid electrolyte layer 101 and the exhaust gas-side electrode 102. The reference gas chamber 105 is formed on an inner side of the solid electrolyte layer 101, and air is introduced into the reference gas chamber 105. The heater 106 is arranged in the reference gas chamber 105, and heats the solid electrolyte layer 101. The sensor element 100 is arranged so as to be exposed to the exhaust gas flowing through the exhaust passage 4. A protective layer may be provided on an outer side of the diffusion control layer 104 to suppress adhesion of liquid or the like to the diffusion control layer 104.

For example, the solid electrolyte layer 101 is formed of a sintered body of an oxygen ion conductive oxide such as $ZrO_2$ (zirconia), $HfO_2$, $ThO_2$, or $Bi_2O_3$ blended with CaO, MgO, $Y_2O_3$, or $Yb_2O_3$ as a stabilizer. The diffusion control layer 104 is formed of a porous sintered body of a heat-proof inorganic substance such as alumina, magnesia, silica, spinel, or mullite. Each of the exhaust gas-side electrode 102 and the air-side electrode 103 is formed of a noble metal having a high catalytic activity, such as Pt (platinum). The materials for the exhaust gas-side electrode 102 and the air-side electrode 103 are not particularly limited as long as oxygen in the exhaust gas guided to the exhaust gas-side electrode 102 via the diffusion control layer 104 can be separated by electrolysis when a desired voltage is applied between the electrodes.

The A/F sensor 10 includes a power supply 107 configured to apply a voltage between the exhaust gas-side electrode 102 and the air-side electrode 103 of the sensor element 100 so that the potential of the air-side electrode 103 is higher than the potential of the exhaust gas-side electrode 102. The magnitude of the voltage to be applied between the exhaust gas-side electrode 102 and the air-side electrode 103 from the power supply 107 is controlled by the ECU 12. The A/F sensor 10 further includes an ammeter 108 configured to measure the magnitude of a current flowing between the exhaust gas-side electrode 102 and the air-side electrode 103 (that is, a current flowing through the solid electrolyte layer 101). A measured value of the ammeter 108 is input to the ECU 12.

Air-Fuel Ratio Detection Operation by A/F Sensor

To detect the air-fuel ratio of the exhaust gas by the A/F sensor 10 constructed as described above, the ECU 12 first causes the heater 106 to heat the sensor element 100 to a temperature equal to or higher than an activation temperature. The "activation temperature" is a temperature at which the solid electrolyte exhibits oxygen ion conductivity. For example, the activation temperature is about 600°C. When the temperature of the sensor element 100 increases to the temperature equal to or higher than the activation temperature, the ECU 12 applies, between the exhaust gas-side electrode 102 and the air-side electrode 103, a voltage at which electrolysis of oxygen may occur.

When the voltage at which electrolysis of oxygen may occur is applied between the exhaust gas-side electrode 102 and the air-side electrode 103 in the state in which the temperature of the sensor element 100 increases to the temperature equal to or higher than the activation temperature, a so-called "oxygen pumping action" occurs. By the oxygen pumping action, oxygen in the exhaust gas is ionized by electrolysis on the exhaust gas-side electrode 102, and ionized oxygen is transferred from the exhaust gas-side electrode 102 to the air-side electrode 103 via the solid electrolyte layer 101. When oxygen ions move from the exhaust gas-side electrode 102 to the air-side electrode 103 by the oxygen pumping action, a current flows between the electrodes. The current flowing between the exhaust gas-side electrode 102 and the air-side electrode 103 (electrode current) tends to increase as the voltage applied between the electrodes (applied voltage) increases. When the amount of exhaust gas that reaches the exhaust gas-side electrode 102 from the exhaust passage 4 is limited by the diffusion control layer 104 and therefore the rate of oxygen consumption along with the oxygen pumping action is higher than the rate of oxygen supply to the exhaust gas-side electrode 102, the oxygen electrolysis reaction is brought into a diffusion-controlled state. In the diffusion-controlled state, a so-called "limiting current characteristic" emerges. In the limiting current characteristic, the electrode current does not increase but is substantially constant even if the applied voltage increases. A range of the applied voltage in which the limiting current characteristic emerges is referred to as a "limiting current range". An electrode current obtained when the voltage in the limiting current range is applied between the exhaust gas-side electrode 102 and the air-side electrode 103 is referred to as a "limiting current".

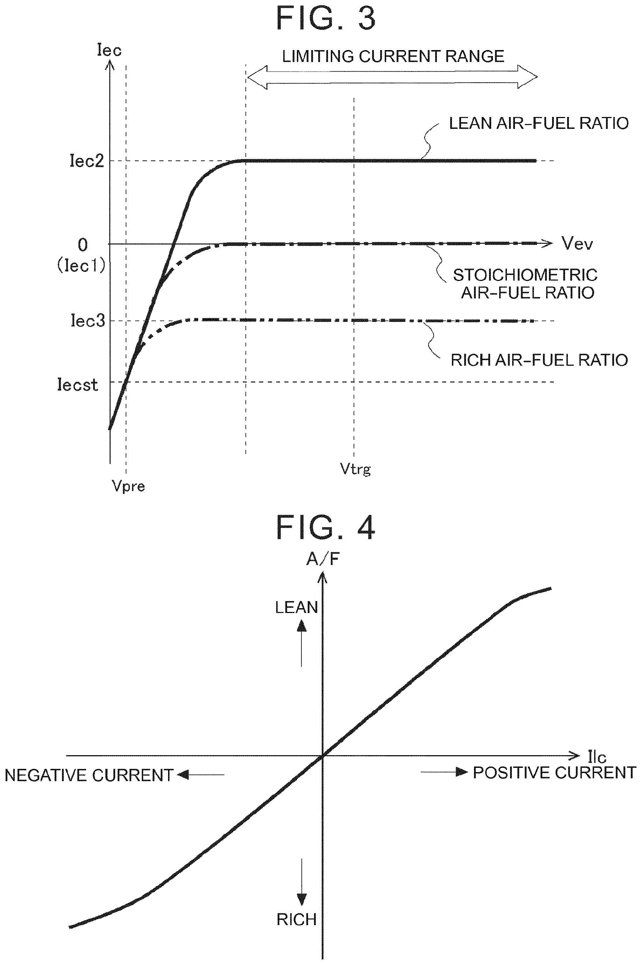

FIG. 3 is a diagram illustrating correlations between the applied voltage (Vev) and the electrode current (Iec) in the A/F sensor 10. In FIG. 3, a continuous line represents a correlation when the air-fuel ratio of the exhaust gas is a lean air-fuel ratio, which is leaner than a stoichiometric air-fuel ratio, a long dashed short dashed line represents a correlation when the air-fuel ratio of the exhaust gas is the stoichiometric air-fuel ratio, and a long dashed double-short dashed line represents a correlation when the air-fuel ratio of the exhaust gas is a rich air-fuel ratio, which is richer than the stoichiometric air-fuel ratio. The A/F sensor 10 of this embodiment is configured such that a limiting current (Ilc0) obtained when the air-fuel ratio of the exhaust gas is the stoichiometric air-fuel ratio is 0 amperes.

As illustrated in FIG. 3, when the applied voltage Vev is lower than the limiting current range, the electrode current Iec increases as the applied voltage Vev increases. When the applied voltage Vev falls within the limiting current range, the electrode current Iec is substantially constant irrespective of the magnitude of the applied voltage Vev. A limiting current obtained when the air-fuel ratio of the exhaust gas is the lean air-fuel ratio (corresponding to Iec2 in FIG. 3) is a positive current larger than a limiting current obtained when the air-fuel ratio of the exhaust gas is the stoichiometric air-fuel ratio (corresponding to Iec1 in FIG. 3). This is because a current flows from a positive terminal of the power supply 107 to a negative terminal of the power supply 107 via the solid electrolyte layer 101 such that surplus oxygen contained in the exhaust gas is ionized on the exhaust gas-side electrode 102 and transferred to the air-side electrode 103. As the air-fuel ratio of the exhaust gas increases (as the lean level increases), the amount of oxygen ions transferred from the exhaust gas-side electrode 102 to the air-side electrode 103 increases. Therefore, the current flowing from the positive terminal of the power supply 107 to the negative terminal of the power supply 107 via the solid electrolyte layer 101 increases along with the increase in the amount of oxygen ions.

A limiting current obtained when the air-fuel ratio of the exhaust gas is the rich air-fuel ratio (corresponding to Iec3 in FIG. 3) is a negative current smaller than the limiting current obtained when the air-fuel ratio of the exhaust gas is the stoichiometric air-fuel ratio (corresponding to Iec1 in FIG. 3). This is because a current flows from the negative terminal of the power supply 107 to the positive terminal of the power supply 107 via the solid electrolyte layer 101 such that oxygen contained in the air in the reference gas chamber 105 is ionized on the air-side electrode 103 and transferred to the exhaust gas-side electrode 102 and oxygen transferred to the exhaust gas-side electrode 102 oxidizes an unburnt fuel component (such as HC or CO) contained in the exhaust gas. As the air-fuel ratio of the exhaust gas decreases (as the rich level increases), the amount of oxygen consumed to oxidize the unburnt fuel component in the exhaust gas increases (that is, the amount of oxygen ions transferred from the air-side electrode 103 to the exhaust gas-side electrode 102 increases). Therefore, the current flowing from the negative terminal to the positive terminal of the power supply 107 via the solid electrolyte layer 101 increases along with the increase in the amount of oxygen.

As illustrated in FIG. 4, the limiting current A/F sensor 10 has a characteristic in which, when the air-fuel ratio of the exhaust gas is the lean air-fuel ratio, the limiting current Ilc is a positive current and increases as the air-fuel ratio of the exhaust gas increases (as the lean level increases) and, when the air-fuel ratio of the exhaust gas is the rich air-fuel ratio, the limiting current Ilc is a negative current and decreases as the air-fuel ratio of the exhaust gas decreases (as the rich level increases). FIG. 4 illustrates a correlation between the limiting current Ilc and the air-fuel ratio of the exhaust gas when a voltage in the limiting current range (for example, about 0.4 volts) is applied between the exhaust gas-side electrode 102 and the air-side electrode 103.

To detect the air-fuel ratio of the exhaust gas, it is appropriate that the ECU 12 acquire the limiting current Ilc by controlling the power supply 107 so that a target voltage Vtrg is applied between the exhaust gas-side electrode 102 and the air-side electrode 103 in the state in which the temperature of the sensor element 100 increases to the temperature equal to or higher than the activation temperature. It is appropriate that the ECU 12 then calculate the air-fuel ratio of the exhaust gas based on the acquired limiting current Ilc and the correlation illustrated in FIG. 4. The "target voltage Vtrg" is a voltage that falls within the limiting current range. The start point of the limiting current range (minimum voltage in the limiting current range) tends to shift to a high-voltage side as the air-fuel ratio of the exhaust gas increases. Therefore, a voltage (for example, about 0.4 volts) higher than that at the start point of a possible maximum air-fuel ratio of the exhaust gas (air-fuel ratio whose lean level is highest) during the operation of the internal combustion engine 1 is set as the target voltage Vtrg. The target voltage Vtrg is set in advance based on results of experiment or simulation.

Failure in A/F Sensor

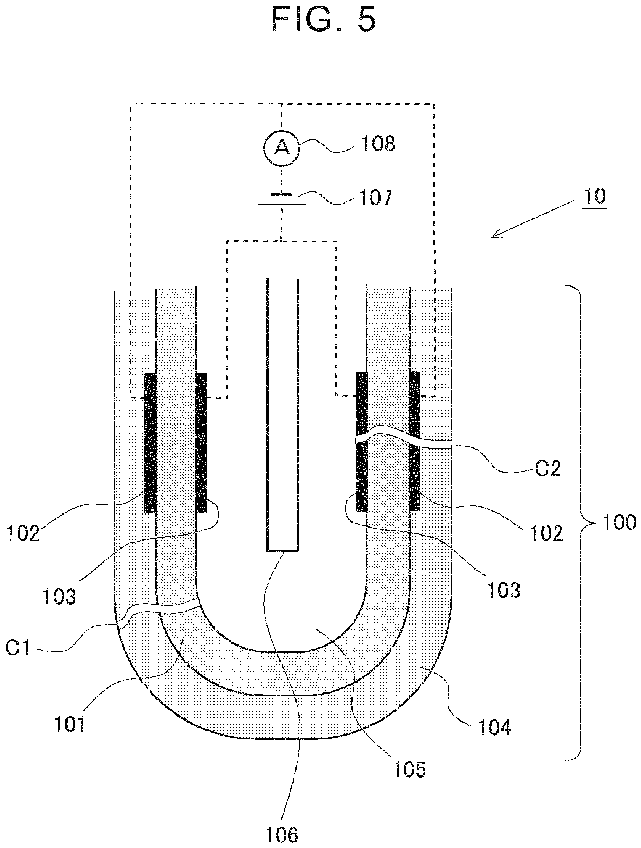

The limiting current A/F sensor 10 described above may fail such that the exhaust gas serving as the detection target gas enters the reference gas chamber 105. For example, the sensor element 100 may be cracked because condensed water generated in the exhaust passage 4 adheres to the sensor element 100. That is, as illustrated in FIG. 5, a crack passing through the solid electrolyte layer 101 and the diffusion control layer 104 (C1 in FIG. 5) or a crack passing through the solid electrolyte layer 101, the diffusion control layer 104, and the electrodes 102, 103 (C2 in FIG. 5) may be generated. When the crack is generated, a part of the exhaust gas flowing through the exhaust passage 4 enters the reference gas chamber 105 via the crack C1 or C2, and is mixed into the air in the reference gas chamber 105.

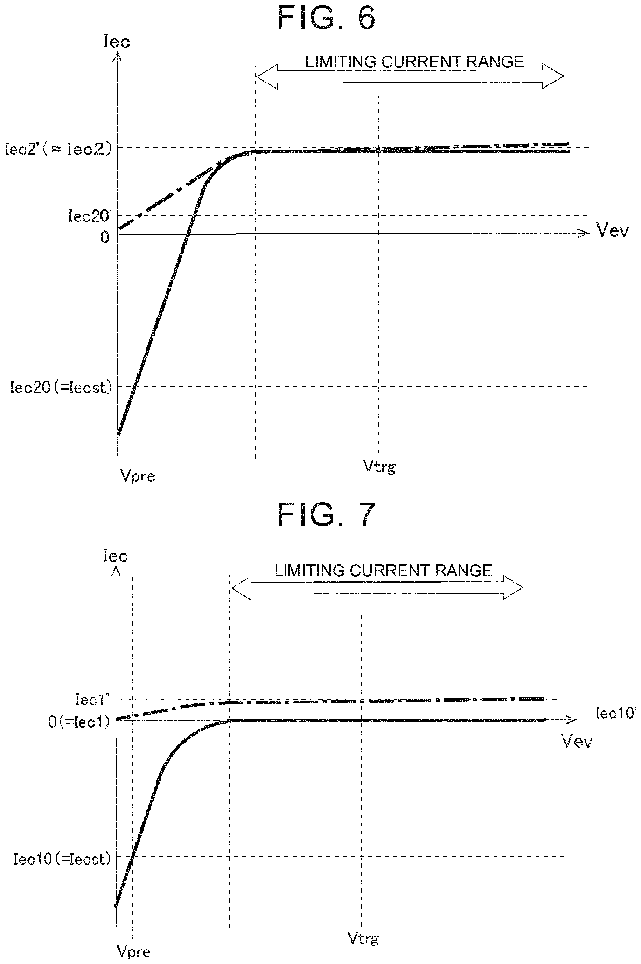

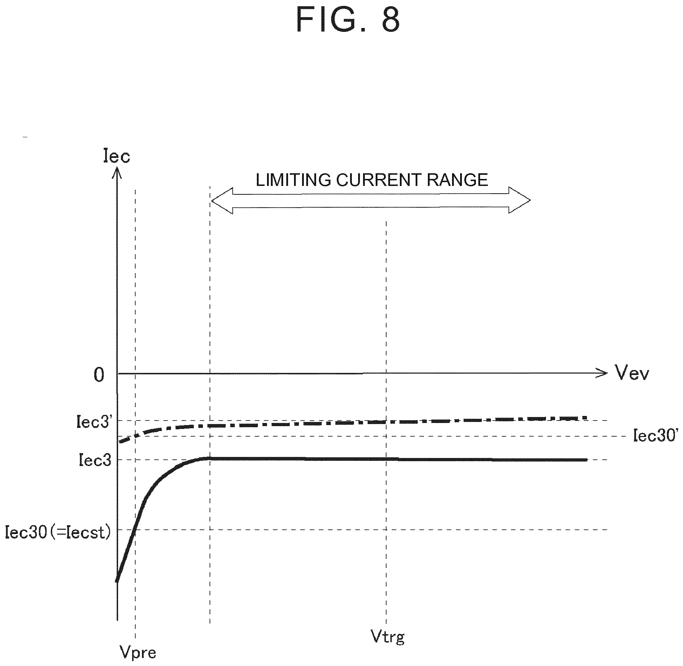

Correlations between the applied voltage Vev and the electrode current Iec when the exhaust gas enters the reference gas chamber 105 due to the cracks or the like are described with reference to FIG. 6 to FIG. 8. FIG. 6 illustrates correlations when the air-fuel ratio of the exhaust gas is the lean air-fuel ratio. FIG. 7 illustrates correlations when the air-fuel ratio of the exhaust gas is the stoichiometric air-fuel ratio. FIG. 8 illustrates correlations when the air-fuel ratio of the exhaust gas is the rich air-fuel ratio. In FIG. 6 to FIG. 8, a continuous line represents a correlation when the exhaust gas does not enter the reference gas chamber 105 (when the A/F sensor 10 does not fail), and a long dashed short dashed line represents a correlation when the exhaust gas enters the reference gas chamber 105 (when the A/F sensor 10 fails).

When the air-fuel ratio of the exhaust gas is the lean air-fuel ratio, as illustrated in FIG. 6, electrode currents obtained when the target voltage Vtrg is applied between the exhaust gas-side electrode 102 and the air-side electrode 103 are substantially equal to each other in the case where the exhaust gas does not enter the reference gas chamber 105 (Iec2 in FIG. 6) and in the case where the exhaust gas enters the reference gas chamber 105 (Iec2' in FIG. 6). The reason is as follows. Even if the exhaust gas containing surplus oxygen and having the lean air-fuel ratio enters the reference gas chamber 105, a difference in oxygen partial pressure between the exhaust gas-side electrode 102 and the air-side electrode 103 (potential difference between the exhaust gas-side electrode 102 and the air-side electrode 103) hardly changes, and therefore the amount of oxygen ions that move from the exhaust gas-side electrode 102 to the air-side electrode 103 hardly changes as well. Thus, when the air-fuel ratio of the exhaust gas is the lean air-fuel ratio, it is difficult to detect the failure in the A/F sensor 10 based on the electrode current obtained when the target voltage Vtrg is applied between the exhaust gas-side electrode 102 and the air-side electrode 103.

When the air-fuel ratio of the exhaust gas is the stoichiometric air-fuel ratio or the rich air-fuel ratio, as illustrated in FIG. 7 and FIG. 8, the electrode current obtained when the target voltage Vtrg is applied between the exhaust gas-side electrode 102 and the air-side electrode 103 is larger (that is, shifted to the positive side) in the case where the exhaust gas enters the reference gas chamber 105 (Iec1' in FIG. 7 and Iec3' in FIG. 8) than the case where the exhaust gas does not enter the reference gas chamber 105 (Iec1 in FIG. 7 and Iec3 in FIG. 8). The reason is as follows. When the exhaust gas having the stoichiometric air-fuel ratio or the rich air-fuel ratio enters the reference gas chamber 105, the difference in the oxygen partial pressure between the exhaust gas-side electrode 102 and the air-side electrode 103 (potential difference between the exhaust gas-side electrode 102 and the air-side electrode 103) decreases, and therefore the amount of oxygen ions transferred from the air-side electrode 103 to the exhaust gas-side electrode 102 decreases. The difference between the electrode current Iec1 or Iec3 obtained when the exhaust gas does not enter the reference gas chamber 105 and the electrode current Iec1' or Iec3' obtained when the exhaust gas enters the reference gas chamber 105 is relatively small, and therefore there is a possibility that the failure in the A/F sensor 10 cannot be detected accurately. Particularly when the amount of exhaust gas that enters the reference gas chamber 105 is infinitesimal, the failure detection accuracy may decrease due to influence of, for example, variations or initial tolerances of the A/F sensor 10. Thus, when the air-fuel ratio of the exhaust gas is the stoichiometric air-fuel ratio or the rich air-fuel ratio, it is difficult to accurately detect the failure in the A/F sensor 10 based on the electrode current obtained when the target voltage Vtrg is applied between the exhaust gas-side electrode 102 and the air-side electrode 103.

Failure Detection for A/F Sensor

In this embodiment, the failure in the A/F sensor 10 is detected focusing on the fact that an electrode current obtained when a predetermined voltage that is a voltage lower than the limiting current range and is a positive voltage is applied between the exhaust gas-side electrode 102 and the air-side electrode 103 exhibits a remarkable difference between the case where the exhaust gas does not enter the reference gas chamber 105 and the case where the exhaust gas enters the reference gas chamber 105.

When the voltage lower than the limiting current range is applied between the exhaust gas-side electrode 102 and the air-side electrode 103 in the state in which the exhaust gas does not enter the reference gas chamber 105, the electrode current decreases as the applied voltage decreases as illustrated in FIG. 3. Particularly when a predetermined relatively low voltage of about 0.1 volts (Vpre in FIG. 3) is applied between the exhaust gas-side electrode 102 and the air-side electrode 103, the electrode current (determination current) is a relatively small negative current. The determination current obtained when the predetermined relatively low voltage Vpre of about 0.1 volts is applied between the exhaust gas-side electrode 102 and the air-side electrode 103 is a substantially constant current (Iecst in FIG. 3) irrespective of the air-fuel ratio of the exhaust gas if the exhaust gas does not enter the reference gas chamber 105.

In the case where the exhaust gas enters the reference gas chamber 105, the electrode current obtained when the voltage that is lower than the limiting current range and is the positive voltage is applied between the exhaust gas-side electrode 102 and the air-side electrode 103 is larger than that in the case where the exhaust gas does not enter the reference gas chamber 105 ("failure characteristic" according to the invention). Particularly when the predetermined relatively low voltage Vpre of about 0.1 volts is applied between the exhaust gas-side electrode 102 and the air-side electrode 103 in the case where the exhaust gas enters the reference gas chamber 105, the determination current exhibits a value remarkably larger than Iecst irrespective of the air-fuel ratio of the exhaust gas. Specifically, when the voltage that is lower than the limiting current range and is the positive voltage is applied between the exhaust gas-side electrode 102 and the air-side electrode 103 in the state in which the air-fuel ratio of the exhaust gas is the lean air-fuel ratio, as illustrated in FIG. 6, the electrode current Iec is larger in the case where the exhaust gas enters the reference gas chamber 105 than the case where the exhaust gas does not enter the reference gas chamber 105. Particularly when the predetermined voltage Vpre is applied between the exhaust gas-side electrode 102 and the air-side electrode 103, a determination current obtained when the exhaust gas does not enter the reference gas chamber 105 (Iec20 (= Iecst) in FIG. 6) is a negative current, but a determination current obtained when the exhaust gas enters the reference gas chamber 105 (Iec20' in FIG. 6) is a positive current. That is, the positive or negative sign of the determination current obtained when the exhaust gas does not enter the reference gas chamber 105 is opposite to that of the determination current obtained when the exhaust gas enters the reference gas chamber 105.

Also when the voltage that is lower than the limiting current range and is the positive voltage is applied between the exhaust gas-side electrode 102 and the air-side electrode 103 in the state in which the air-fuel ratio of the exhaust gas is the stoichiometric air-fuel ratio, as illustrated in FIG. 7, the electrode current Iec is larger in the case where the exhaust gas enters the reference gas chamber 105 than the case where the exhaust gas does not enter the reference gas chamber 105. When the predetermined voltage Vpre is applied between the exhaust gas-side electrode 102 and the air-side electrode 103, a determination current obtained when the exhaust gas does not enter the reference gas chamber 105 (Iec10 (= Iecst) in FIG. 7) is a negative current, but a determination current obtained when the exhaust gas enters the reference gas chamber 105 (Iec10' in FIG. 7) is a positive current.

Also when the voltage that is lower than the limiting current range and is the positive voltage is applied between the exhaust gas-side electrode 102 and the air-side electrode 103 in the state in which the air-fuel ratio of the exhaust gas is the rich air-fuel ratio, as illustrated in FIG. 8, the electrode current Iec is larger in the case where the exhaust gas enters the reference gas chamber 105 than the case where the exhaust gas does not enter the reference gas chamber 105. When the predetermined voltage Vpre is applied between the exhaust gas-side electrode 102 and the air-side electrode 103, a determination current obtained when the exhaust gas enters the reference gas chamber 105 (Iec30' in FIG. 8) is a negative current similarly to a determination current obtained when the exhaust gas does not enter the reference gas chamber 105 (Iec30 (= Iecst) in FIG. 8), but is remarkably larger than the determination current obtained when the exhaust gas does not enter the reference gas chamber 105 (Iec30 in FIG. 8).

The difference in the determination current between the case where the exhaust gas does not enter the reference gas chamber 105 and the case where the exhaust gas enters the reference gas chamber 105 tends to be larger in the case where the air-fuel ratio of the exhaust gas is high than the case where the air-fuel ratio of the exhaust gas is low.

The failure characteristic illustrated in FIG. 6 to FIG. 8 may emerge for the following reason. The difference in the oxygen partial pressure between the exhaust gas-side electrode 102 and the air-side electrode 103 in the case where the exhaust gas enters the reference gas chamber 105 is smaller than that in the case where the exhaust gas does not enter the reference gas chamber 105. Therefore, the amount of oxygen ions transferred from the air-side electrode 103 to the exhaust gas-side electrode 102 decreases. Thus, the current easily flows from the air-side electrode 103 to the exhaust gas-side electrode 102 when the voltage is applied between the exhaust gas-side electrode 102 and the air-side electrode 103.

In this embodiment, the voltage at which the failure characteristic emerges in the event of the failure in the A/F sensor 10 due to the entry of the exhaust gas into the reference gas chamber 105 is set to the predetermined voltage Vpre. Determination is made as to whether the A/F sensor 10 fails based on the determination current that is the electrode current obtained when the predetermined voltage Vpre is applied between the exhaust gas-side electrode 102 and the air-side electrode 103. Specifically, determination is made that the A/F sensor 10 fails due to the entry of the exhaust gas into the reference gas chamber 105 if the determination current measured by the ammeter 108 when the predetermined voltage Vpre set as described above is applied between the exhaust gas-side electrode 102 and the air-side electrode 103 is larger than a predetermined threshold. The "predetermined threshold" is a value obtained by adding a predetermined margin to Iecst.

Processing Flow

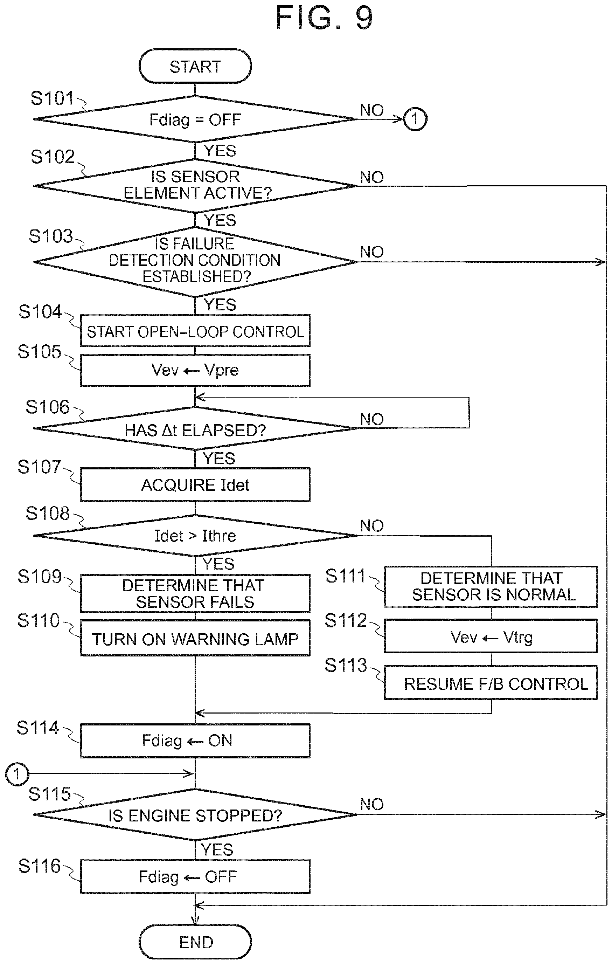

A flow of failure detection processing of this embodiment is described with reference to FIG. 9. FIG. 9 is a flowchart illustrating a processing routine to be executed by the ECU 12 in the failure detection processing according to this embodiment. The processing routine illustrated in FIG. 9 is executed in a predetermined cycle during an operating period of the internal combustion engine 1, and is stored in advance in, for example, the ROM of the ECU 12.

In the processing routine of FIG. 9, the ECU 12 first determines in S101 whether a failure detection flag Fdiag is ON. The failure detection flag Fdiag is ON when the failure detection processing is terminated, and is OFF when the operation of the internal combustion engine 1 is stopped (for example, when an ignition switch is changed from ON to OFF). When the determination is "no" in S101 (failure detection flag Fdiag = ON), the ECU 12 proceeds to S115 by skipping processing of S102 to S114. When the determination is "yes" in S101 (failure detection flag Fdiag = OFF), the ECU 12 proceeds to S102.

In S102, the ECU 12 determines whether the sensor element 100 of the A/F sensor 10 is active. Specifically, the ECU 12 calculates the temperature of the sensor element 100 from an impedance obtained when a high-frequency voltage is applied between the exhaust gas-side electrode 102 and the air-side electrode 103, and determines that the sensor element 100 is active when the temperature is equal to or higher than the activation temperature. The temperature of the sensor element 100 may be detected by separately providing a temperature sensor. When the determination is "no" in S102, the ECU 12 terminates the execution of the processing routine. When the determination is "yes" in S102, the ECU 12 proceeds to S103.

In S103, the ECU 12 determines whether a failure detection condition is established. For example, the "failure detection condition" is that the flow rate of the exhaust gas is equal to or higher than a predetermined flow rate (or a state in which the flow rate of the exhaust gas is equal to or higher than the predetermined flow rate continues for a predetermined time or longer), or that warm-up of the internal combustion engine 1 is completed. The "predetermined flow rate" is a flow rate of the exhaust gas that may enter the reference gas chamber 105 even if the crack of the sensor element 100 is minute as long as the flow rate of the exhaust gas is equal to or higher than the predetermined flow rate. The predetermined flow rate is set in advance based on results of experiment or simulation. When the determination is "no" in S103, the ECU 12 terminates the execution of the processing routine. When the determination is "yes" in S103, the ECU 12 proceeds to S104.

In S104, the ECU 12 stops so-called "air-fuel ratio feedback control (F/B control)" for correcting a target injection amount based on the measured value of the A/F sensor 10, and starts open-loop control for determining the target injection amount based on a predetermined target air-fuel ratio instead. The reason is as follows. In the failure detection processing, the applied voltage of the A/F sensor 10 (voltage applied between the exhaust gas-side electrode 102 and the air-side electrode 103) is changed to the voltage lower than the limiting current range. Therefore, the A/F sensor 10 cannot accurately measure the air-fuel ratio of the exhaust gas. The "predetermined target air-fuel ratio" need not be a specific air-fuel ratio, but may be set depending on, for example, an operation status of the internal combustion engine 1. If the A/F sensor 10 subjected to the failure detection processing is the second A/F sensor 10b arranged downstream of the catalyst casing 9, the processing of S104 may be avoided.

In S105, the ECU 12 controls the power supply 107 so as to change the applied voltage Vev of the A/F sensor 10 from the target voltage Vtrg to the predetermined voltage Vpre. As described above, the predetermined voltage Vpre is the applied voltage at which the failure characteristic emerges in the event of the failure due to the entry of the exhaust gas into the reference gas chamber 105. More specifically, the predetermined voltage Vpre is the applied voltage at which the positive or negative sign of the determination current obtained when the exhaust gas enters the reference gas chamber 105 is opposite to that of the determination current obtained when the exhaust gas does not enter the reference gas chamber 105 as illustrated in FIG. 6 if the air-fuel ratio of the exhaust gas is the lean air-fuel ratio, which is leaner than the stoichiometric air-fuel ratio. In this example, the applied voltage (for example, 0.1 volts) at which the determination current obtained when the exhaust gas does not enter the reference gas chamber 105 exhibits a substantially constant value (Iecst in FIG. 3) irrespective of the air-fuel ratio of the exhaust gas is set to the predetermined voltage Vpre.

In S106, the ECU 12 determines whether a predetermined time Δt elapses after the applied voltage Vev is changed from the target voltage Vtrg to the predetermined voltage Vpre. The "predetermined time Δt" is a time required from a timing when the applied voltage Vev is changed to a timing when the change in the applied voltage Vev is reflected in the electrode current Iec. The predetermined time Δt is set in advance based on results of experiment or simulation. When the determination is "no" in S106, the ECU 12 repeats the processing of S106. When the determination is "yes" in S106, the ECU 12 proceeds to S107.

In S107, the ECU 12 reads the measured value of the ammeter 108 to acquire a determination current Idet that is the electrode current obtained when the predetermined voltage Vpre is applied between the exhaust gas-side electrode 102 and the air-side electrode 103.

In S108, the ECU 12 determines whether the determination current Idet acquired in S107 is larger than a predetermined threshold Ithre. As described above, the "predetermined threshold Ithre" is the value obtained by adding the predetermined margin to the determination current obtained when the exhaust gas does not enter the reference gas chamber 105 (corresponding to Iecst in FIG. 3).

When the determination is "yes" in S108 (Idet > Ithre), the ECU 12 proceeds to S109, and determines that the A/F sensor 10 fails due to the entry of the exhaust gas into the reference gas chamber 105. Subsequently, the ECU 12 proceeds to S110, and performs, for example, processing of turning ON a warning lamp so as to notify a driver of the vehicle that the A/F sensor 10 fails.

When the determination is "no" in S108 (Idet ≤ Ithre), the ECU 12 proceeds to S111, and determines that the failure in the A/F sensor 10 due to the entry of the exhaust gas into the reference gas chamber 105 does not occur (the A/F sensor 10 is normal). Subsequently, the ECU 12 proceeds to S112, and controls the power supply 107 so as to return the applied voltage Vev of the A/F sensor 10 from the predetermined voltage Vpre to the target voltage Vtrg. Then, the ECU 12 stops the open-loop control for determining the target injection amount based on the predetermined target air-fuel ratio, and resumes the air-fuel ratio feedback control (F/B control) for correcting the target injection amount based on the measured value of the A/F sensor 10 instead (S113). If the A/F sensor 10 subjected to the failure detection processing is the second A/F sensor 10b arranged downstream of the catalyst casing 9 and if the processing of S104 is not performed, the processing of S113 is not performed.

When the ECU 12 finishes executing the processing of S110 or S113, the ECU 12 proceeds to S114, and changes the failure detection flag Fdiag from OFF to ON. In S115, the ECU 12 determines whether the operation of the internal combustion engine 1 is stopped. Specifically, the ECU 12 determines that the operation of the internal combustion engine 1 is stopped when the ignition switch is changed from ON to OFF. When the determination is "no" in S115, the ECU 12 terminates the execution of the processing routine. When the determination is "yes" in S115, the ECU 12 proceeds to S116, and changes the failure detection flag Fdiag from ON to OFF. Then, the ECU 12 terminates the execution of the processing routine.

When the failure detection processing for the A/F sensor 10 is performed through the processing routine described above, the failure in the A/F sensor 10 due to the entry of the exhaust gas into the reference gas chamber 105 can accurately be detected irrespective of the air-fuel ratio of the exhaust gas (concentration of oxygen contained in the exhaust gas).

In the processing routine of FIG. 9, description is given of the example in which the applied voltage (for example, 0.1 volts) at which the determination current obtained when the exhaust gas does not enter the reference gas chamber 105 exhibits a substantially constant value (Iecst in FIG. 3) irrespective of the air-fuel ratio of the exhaust gas is set to the predetermined voltage Vpre. The invention is not limited to this example. The predetermined voltage Vpre may be any applied voltage at which the failure characteristic emerges. If the predetermined voltage Vpre is set to an applied voltage higher than 0.1 volts, the determination current obtained when the exhaust gas does not enter the reference gas chamber 105 may vary depending on the air-fuel ratio of the exhaust gas. In this case, the predetermined threshold may be set to a value larger than a possible maximum value of the determination current Idet obtained when the exhaust gas does not enter the reference gas chamber 105. If the A/F sensor 10 subjected to the failure detection processing is the first A/F sensor 10a arranged upstream of the catalyst casing 9, the air-fuel ratio of the exhaust gas flowing into the first A/F sensor 10a can be estimated from the target air-fuel ratio. Therefore, the predetermined threshold may be set to a value that varies depending on the air-fuel ratio of the exhaust gas. For example, the predetermined threshold may be set to a value that increases as the air-fuel ratio of the exhaust gas increases.

Second Embodiment

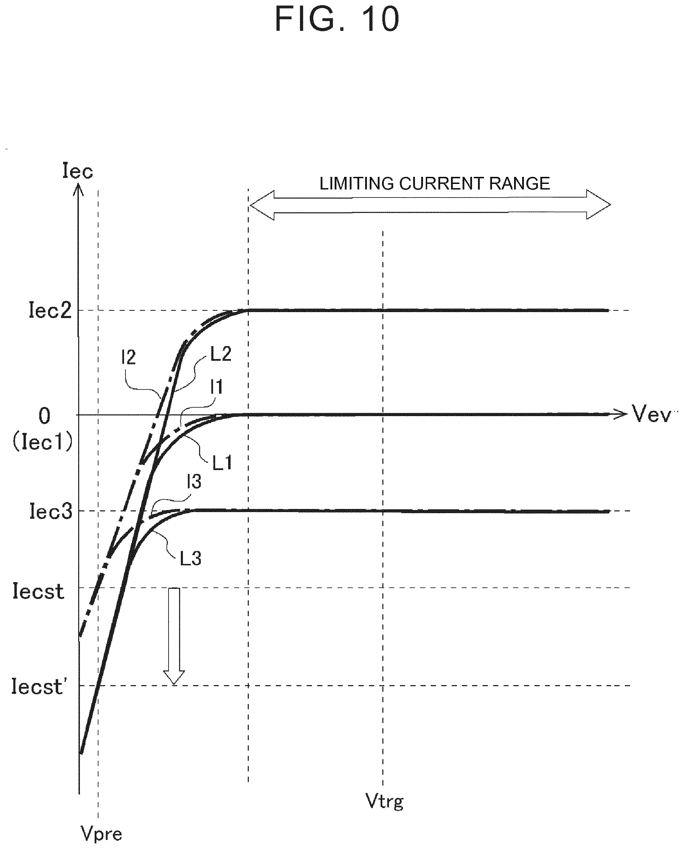

Next, a second embodiment of the invention is described with reference to FIG. 10 to FIG. 12. Description is given of structures different from those of the first embodiment described above, and description of similar structures is omitted.

This embodiment differs from the first embodiment in that the heater 106 heats the sensor element 100 to a temperature equal to or higher than a predetermined temperature that is higher than the activation temperature when the predetermined voltage is applied between the exhaust gas-side electrode 102 and the air-side electrode 103.

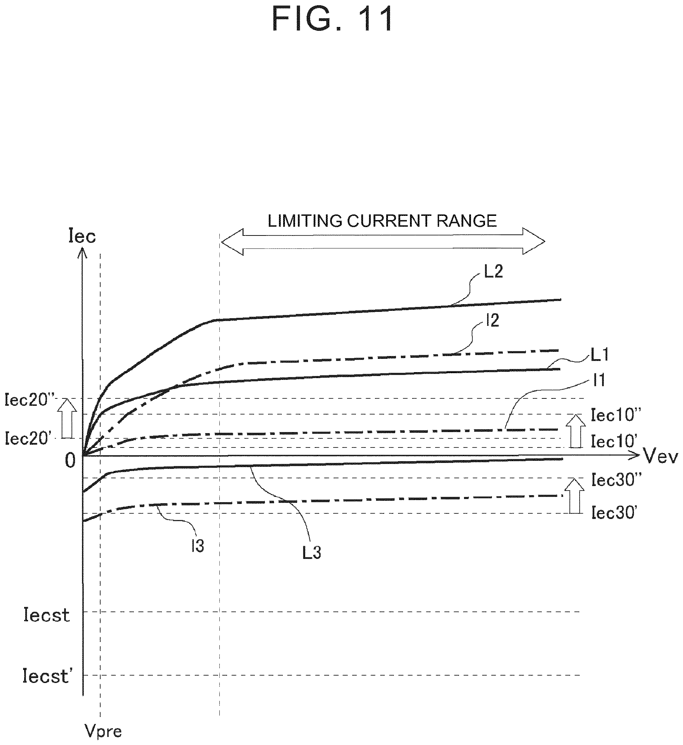

FIG. 10 and FIG. 11 illustrate correlations between the applied voltage Vev and the electrode current Iec when the sensor element 100 is heated to the temperature equal to or higher than the predetermined temperature (for example, 750°C to 800°C). FIG. 10 illustrates correlations between the applied voltage Vev and the electrode current Iec when the exhaust gas does not enter the reference gas chamber 105. FIG. 11 illustrates correlations between the applied voltage Vev and the electrode current Iec when the exhaust gas enters the reference gas chamber 105. In FIG. 10 and FIG. 11, a continuous line represents a correlation when the sensor element 100 is heated to the temperature equal to or higher than the predetermined temperature, and a long dashed short dashed line represents a correlation when the temperature of the sensor element 100 is the activation temperature (for example, about 600°C). In FIG. 10 and FIG. 11, lines L1 and I1 represent correlations when the air-fuel ratio of the exhaust gas is the stoichiometric air-fuel ratio, lines L2 and I2 represent correlations when the air-fuel ratio of the exhaust gas is the lean air-fuel ratio, and lines L3 and I3 represent correlations when the air-fuel ratio of the exhaust gas is the rich air-fuel ratio.

As represented by the lines L1 and I1 in FIG. 10, if the exhaust gas does not enter the reference gas chamber 105 when the target voltage Vtrg in the limiting current range is applied between the exhaust gas-side electrode 102 and the air-side electrode 103 in the state in which the air-fuel ratio of the exhaust gas is the stoichiometric air-fuel ratio, the electrode currents are equal to each other (Iec1 in FIG. 10) in the case where the temperature of the sensor element 100 is equal to or higher than the predetermined temperature and in the case where the temperature of the sensor element 100 is the activation temperature. If the exhaust gas does not enter the reference gas chamber 105 when the predetermined voltage Vpre is applied between the exhaust gas-side electrode 102 and the air-side electrode 103 in the state in which the air-fuel ratio of the exhaust gas is the stoichiometric air-fuel ratio, the electrode current obtained when the temperature of the sensor element 100 is equal to or higher than the predetermined temperature (Iecst' in FIG. 10) is smaller than the electrode current obtained when the temperature of the sensor element 100 is the activation temperature (Iecst in FIG. 10). The reason is as follows. In the limiting current range, influence exerted on the electrode current by the diffusion control action for the exhaust gas by the diffusion control layer 104 is dominant over influence exerted on the electrode current by the magnitude of a resistance between the exhaust gas-side electrode 102 and the air-side electrode 103 (may hereinafter be referred to as "inter-electrode resistance"). In a voltage range lower than the limiting current range, the influence exerted on the electrode current by the magnitude of the inter-electrode resistance is dominant over the influence exerted on the electrode current by the diffusion control action for the exhaust gas by the diffusion control layer 104. That is, the magnitude of the inter-electrode resistance is smaller in the case where the temperature of the sensor element 100 is high than the case where the temperature of the sensor element 100 is low. Therefore, the amount of oxygen ions that move between the exhaust gas-side electrode 102 and the air-side electrode 103 increases in the voltage range lower than the limiting current range. Thus, the electrode current obtained when the predetermined voltage Vpre is applied between the exhaust gas-side electrode 102 and the air-side electrode 103 is shifted to a negative-current side.

As represented by the lines L2, I2, L3, and I3 in FIG. 10, if the exhaust gas does not enter the reference gas chamber 105 when the target voltage Vtrg in the limiting current range is applied between the exhaust gas-side electrode 102 and the air-side electrode 103 in the state in which the air-fuel ratio of the exhaust gas is the lean air-fuel ratio or the rich air-fuel ratio, the electrode currents are equal to each other (Iec2 or Iec3 in FIG. 10) in the case where the temperature of the sensor element 100 is equal to or higher than the predetermined temperature and in the case where the temperature of the sensor element 100 is the activation temperature. If the exhaust gas does not enter the reference gas chamber 105 when the predetermined voltage Vpre is applied between the exhaust gas-side electrode 102 and the air-side electrode 103 in the state in which the air-fuel ratio of the exhaust gas is the lean air-fuel ratio or the rich air-fuel ratio, the electrode current obtained when the temperature of the sensor element 100 is equal to or higher than the predetermined temperature (Iecst' in FIG. 10) is smaller than the electrode current obtained when the temperature of the sensor element 100 is the activation temperature (Iecst in FIG. 10).

As represented by the lines L1 and I1 in FIG. 11, if the exhaust gas enters the reference gas chamber 105 when the predetermined voltage Vpre is applied between the exhaust gas-side electrode 102 and the air-side electrode 103 in the state in which the air-fuel ratio of the exhaust gas is the stoichiometric air-fuel ratio, the electrode current obtained when the temperature of the sensor element 100 is equal to or higher than the predetermined temperature (Iec10" in FIG. 11) is larger than the electrode current obtained when the temperature of the sensor element 100 is the activation temperature (Iec10' in FIG. 11). As represented by the lines L2 and I2 in FIG. 11, if the exhaust gas enters the reference gas chamber 105 when the predetermined voltage Vpre is applied between the exhaust gas-side electrode 102 and the air-side electrode 103 in the state in which the air-fuel ratio of the exhaust gas is the lean air-fuel ratio, the electrode current obtained when the temperature of the sensor element 100 is equal to or higher than the predetermined temperature (Iec20" in FIG. 11) is larger than the electrode current obtained when the temperature of the sensor element 100 is the activation temperature (Iec20' in FIG. 11). As represented by L3 and I3 in FIG. 11, if the exhaust gas enters the reference gas chamber 105 when the predetermined voltage Vpre is applied between the exhaust gas-side electrode 102 and the air-side electrode 103 in the state in which the air-fuel ratio of the exhaust gas is the rich air-fuel ratio, the electrode current obtained when the temperature of the sensor element 100 is equal to or higher than the predetermined temperature (Iec30" in FIG. 11) is larger than the electrode current obtained when the temperature of the sensor element 100 is the activation temperature (Iec30' in FIG. 11). As is apparent from those facts, if the exhaust gas enters the reference gas chamber 105 when the predetermined voltage Vpre is applied between the exhaust gas-side electrode 102 and the air-side electrode 103, the electrode current is larger (shifted to a positive-current side) in the case where the temperature of the sensor element 100 is equal to or higher than the predetermined temperature than the case where the temperature of the sensor element 100 is the activation temperature irrespective of the air-fuel ratio of the exhaust gas.

According to the characteristics illustrated in FIG. 10 and FIG. 11, if the temperature of the sensor element 100 is increased to the temperature equal to or higher than the predetermined temperature when the predetermined voltage Vpre is applied between the exhaust gas-side electrode 102 and the air-side electrode 103, the difference in the determination current between the case where the exhaust gas enters the reference gas chamber 105 and the case where the exhaust gas does not enter the reference gas chamber 105 can be increased. As a result, even if a minute crack is generated in the sensor element 100, the failure in the A/F sensor 10 due to the crack can be detected accurately.

Processing Flow

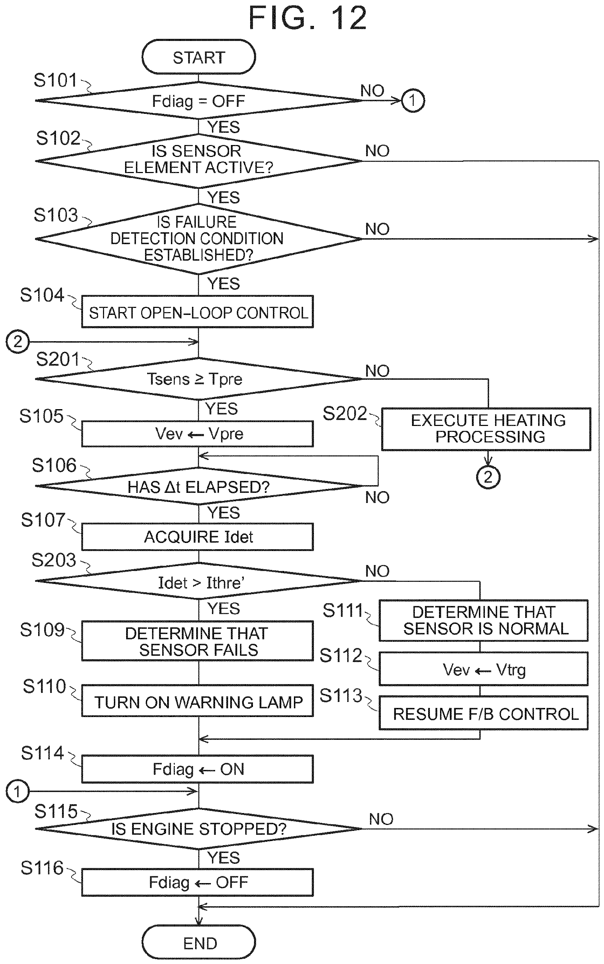

A flow of failure detection processing of this embodiment is described with reference to FIG. 12. FIG. 12 is a flowchart illustrating a processing routine to be executed by the ECU 12 in the failure detection processing according to this embodiment. In FIG. 12, processing operations similar to those of the processing routine illustrated in FIG. 9 are represented by the same reference symbols.

In the processing routine of FIG. 12, the ECU 12 proceeds to S201 after the processing of S104 is executed, and determines whether a temperature Tsens of the sensor element 100 is equal to or higher than a predetermined temperature Tpre. As described above, the "predetermined temperature Tpre" is the temperature of the sensor element 100 that is higher than the activation temperature of the sensor element 100, and is the temperature at which the difference in the determination current between the case where the exhaust gas enters the reference gas chamber 105 and the case where the exhaust gas does not enter the reference gas chamber 105 is larger than that in the case where the temperature of the sensor element 100 is the activation temperature. For example, the predetermined temperature Tpre is 750°C to 800°C. As described in the first embodiment, the temperature Tsens of the sensor element 100 may be calculated from the impedance obtained when the high-frequency voltage is applied between the exhaust gas-side electrode 102 and the air-side electrode 103, or may be measured by the temperature sensor that is provided separately.

When the determination is "no" in S201 (Tsens < Tpre), the ECU 12 proceeds to S202, and controls the heater 106 so as to increase the temperature of the sensor element 100 to the temperature equal to or higher than the predetermined temperature Tpre (heating processing). For example, the ECU 12 may control the energization amount of the heater 106 based on a difference between the temperature Tsens of the sensor element 100 and the predetermined temperature Tpre. That is, the energization amount of the heater 106 may be set larger in a case where the difference between the temperature Tsens of the sensor element 100 and the predetermined temperature Tpre is large than a case where the difference is small. If the temperature of the sensor element 100 is excessively increased to a temperature equal to or higher than 900°C, the measurement accuracy of the A/F sensor 10 may decrease, or the sensor element 100 may thermally deteriorate. In the heating processing, feedback control may be performed for the energization amount of the heater 106 so that the temperature of the sensor element 100 does not exceed 900°C.

When the ECU 12 finishes executing the processing of S202, the ECU 12 returns to the processing of S201. When the determination is "yes" in the processing of S201 (Tsens ≥ Tpre), the ECU 12 sequentially executes the processing of S105 to the processing of S107. Immediately after the sensor element 100 is heated to the predetermined temperature Tpre through the heating processing, there is a possibility that the change in the temperature of the sensor element 100 (change in the inter-electrode resistance) is not reflected in the electrode current Iec. Therefore, the ECU 12 may execute the processing of S105 after an elapse of a predetermined waiting time (for example, about 2 sec) from the timing when the determination is "yes" in S201.

In the processing routine of FIG. 12, when the ECU 12 finishes executing the processing of S107, the ECU 12 executes processing of S203 in place of S108 of the processing routine of FIG. 9. In S203, the ECU 12 determines whether the determination current Idet acquired in S107 is larger than a predetermined threshold Ithre'. The "predetermined threshold Ithre'" is a value obtained by adding a predetermined margin to the determination current obtained when the exhaust gas does not enter the reference gas chamber 105 and the temperature of the sensor element 100 is equal to or higher than the predetermined temperature Tpre (corresponding to Iecst' in FIG. 10). When the determination is "yes" in S203 (Idet > Ithre'), the ECU 12 proceeds to S109. When the determination is "no" in S203 (Idet ≤ Ithre'), the ECU 12 proceeds to S111.

When the failure detection processing for the A/F sensor 10 is performed through the processing routine of FIG. 12, the failure in the A/F sensor 10 due to the entry of the exhaust gas into the reference gas chamber 105 can be detected more accurately irrespective of the air-fuel ratio of the exhaust gas. In particular, the failure in the A/F sensor 10 can be detected more securely even if the exhaust gas enters the reference gas chamber 105 due to a minute crack in the sensor element 100.

Other Embodiments

In the first and second embodiments described above, description is given of the example in which the failure in the A/F sensor 10 due to the entry of the exhaust gas into the reference gas chamber 105 is detected by comparing the predetermined threshold Ithre with the electrode current (determination current Idet) measured by the ammeter 108 when the predetermined voltage Vpre is applied between the exhaust gas-side electrode 102 and the air-side electrode 103. The failure in the A/F sensor 10 due to the entry of the exhaust gas into the reference gas chamber 105 may be detected based on the magnitude of a resistance between the exhaust gas-side electrode 102 and the air-side electrode 103 when the predetermined voltage Vpre is applied between the electrodes.

Specifically, the ECU 12 first calculates the resistance between the exhaust gas-side electrode 102 and the air-side electrode 103 (may hereinafter be referred to as "determination resistance Rdet") by substituting the predetermined voltage Vpre and the determination current Idet into Expression (1).

$$Rdet = Vpre / (-Idet) \qquad (1)$$

When the air-fuel ratio of the exhaust gas is the stoichiometric air-fuel ratio or the lean air-fuel ratio, the positive or negative sign of the determination current Idet obtained when the exhaust gas does not enter the reference gas chamber 105 is opposite to that of the determination current Idet obtained when the exhaust gas enters the reference gas chamber 105. Therefore, a determination resistance obtained when the exhaust gas enters the reference gas chamber 105 is a negative resistance smaller than a determination resistance obtained when the exhaust gas does not enter the reference gas chamber 105. When the air-fuel ratio of the exhaust gas is the rich air-fuel ratio, the positive or negative sign of the determination current Idet obtained when the exhaust gas does not enter the reference gas chamber 105 is not opposite to that of the determination current Idet obtained when the exhaust gas enters the reference gas chamber 105. However, the determination current Idet obtained when the exhaust gas enters the reference gas chamber 105 is extremely larger than the determination current Idet obtained when the exhaust gas does not enter the reference gas chamber 105. Thus, the determination resistance obtained when the exhaust gas enters the reference gas chamber 105 is extremely larger than the determination resistance obtained when the exhaust gas does not enter the reference gas chamber 105.

Determination may be made that the A/F sensor 10 fails due to the entry of the exhaust gas into the reference gas chamber 105 when the determination resistance Rdet is larger than a predetermined upper limit value or when the determination resistance Rdet is smaller than a predetermined lower limit value (= 0). As described above, the "predetermined upper limit value" is a possible minimum value of the determination resistance Rdet obtained when the exhaust gas enters the reference gas chamber 105 and when the air-fuel ratio of the exhaust gas is the rich air-fuel ratio. The predetermined upper limit value may be determined in advance based on results of experiment or simulation.

Others

In the embodiments described above, description is given of the example in which the invention is applied to the A/F sensor configured to detect the concentration of oxygen contained in the exhaust gas from the internal combustion engine. The gas sensor to which the invention is applied is not limited to this example. The invention is also applicable to a limiting current gas sensor configured to detect the concentration of, for example, a nitrogen oxide ($NO_X$), a sulfur oxide ($SO_X$), water ($H_2O$), or carbon dioxide ($CO_2$) in the exhaust gas by using the oxygen pumping action. The gas to be subjected to detection by the gas sensor to which the invention is applied (detection target gas) is not limited to the exhaust gas from the internal combustion engine, but may be any gas discharged in manufacturing processes for various products.

**Claims**

1. A failure detection apparatus for a gas sensor (10), the gas sensor (10) including a sensor element (100) including a solid electrolyte (101) having oxide ion conductivity, a pair of electrodes (102, 103) attached to the solid electrolyte (101), and a diffusion control portion (104) that controls a rate of diffusion of detection target gas to guide the detection target gas to one of the pair of electrodes (102, 103), the gas sensor (10) being a limiting current gas sensor configured to detect a concentration of a predetermined component contained in the detection target gas, the failure detection apparatus comprising an electronic control unit (12) configured to:

    perform control so that a predetermined voltage is applied between the pair of electrodes (102, 103), the predetermined voltage being a positive voltage and being a voltage lower than a limiting current range when the gas sensor (10) does not fail;
    acquire a determination current that is a current flowing between the pair of electrodes (102, 103) when the predetermined voltage is applied between the pair of electrodes (102, 103); and
    determine whether the gas sensor (10) fails based on the acquired determination current.

2. The failure detection apparatus according to claim 1, wherein:

    the gas sensor (10) is arranged in an exhaust passage (4) of an internal combustion engine (1), and is configured to detect a concentration of oxygen serving as the predetermined component, which is contained in exhaust gas serving as the detection target gas;
    the electronic control unit (12) is configured to determine whether the gas sensor (10) fails based on a failure characteristic; and
    the failure characteristic is a characteristic in which the determination current is larger in a case where the gas sensor (10) fails than a case where the gas sensor (10) does not fail.

3. The failure detection apparatus according to claim 2, wherein:

    one electrode out of the pair of electrodes (102, 103) is arranged so as to face a reference gas chamber into which air is introduced;
    the other electrode out of the pair of electrodes (102, 103) is arranged so as to be exposed to the exhaust gas that serves as the detection target gas and is introduced via the diffusion control portion (104);
    the electronic control unit (12) is configured such that a voltage at which the failure characteristic emerges in an event of failure is applied between the pair of electrodes as the predetermined voltage; and
    the failure is failure due to entry of the exhaust gas into the reference gas chamber.

4. The failure detection apparatus according to claim 3, wherein
the predetermined voltage is a voltage at which, when an air-fuel ratio of the exhaust gas is a lean air-fuel ratio, a positive or negative sign of the determination current obtained when the exhaust gas does not enter the reference gas chamber is opposite to a positive or negative sign of the determination current obtained when the exhaust gas enters the reference gas chamber, and
the learn air-fuel ratio is learner than a stoichiometric air-fuel ratio.

5. The failure detection apparatus according to claim 4, wherein the predetermined voltage is 0.1 volts.

6. The failure detection apparatus according to any one of claims 3 to 5, wherein the electronic control unit (12) is configured to determine that the gas sensor (10) fails when the acquired determination current is larger than a predetermined threshold.

7. The failure detection apparatus according to any one of claims 3 to 5, wherein:

    the electronic control unit (12) is configured to calculate a resistance value between the pair of electrodes (102, 103) based on the acquired determination current and the predetermined voltage; and
    the electronic control unit (12) is configured to determine that the gas sensor (10) fails when the calculated resistance value is larger than a predetermined upper limit value or when the calculated resistance value is smaller than a predetermined lower limit value.

8. The failure detection apparatus according to any one of claims 1 to 5, wherein:

the gas sensor (10) further includes a heater configured to heat the sensor element (100); and the electronic control unit (12) is configured to control the heater so as to increase a temperature of the sensor element (100) to a temperature equal to or higher than a predetermined temperature, which is higher than an activation temperature of the sensor element (100), when the predetermined voltage is applied between the pair of electrodes (102, 103).

9. A failure detection method for a gas sensor (10), the gas sensor (10) including a sensor element (100) including a solid electrolyte (101) having oxide ion conductivity, a pair of electrodes (102, 103) attached to the solid electrolyte (101), and a diffusion control portion (104) that controls a rate of diffusion of detection target gas to guide the detection target gas to one of the pair of electrodes (102, 103), the gas sensor (10) being a limiting current gas sensor configured to detect a concentration of a predetermined component contained in the detection target gas, the failure detection method comprising:

   performing, by an electronic control unit (12), control so that a predetermined voltage is applied between the pair of electrodes (102, 103), the predetermined voltage being a positive voltage and being a voltage lower than a limiting current range when the gas sensor (10) does not fail;
   acquiring, by the electronic control unit (12), a determination current that is a current flowing between the pair of electrodes (102, 103) when the predetermined voltage is applied between the pair of electrodes (102, 103); and
   determining, by the electronic control unit (12), whether the gas sensor (10) fails based on the determination current.

# FIG. 1

# FIG. 2

EXHAUST
GAS

EXHAUST
GAS

102

103

101

102

103

104

105

106

100

10

108

107

# FIG. 3

# FIG. 4

# FIG. 5

# FIG. 6

# FIG. 7

# FIG. 8

# FIG. 9

```
                    ┌──────────┐
                    │  START   │
                    └──────────┘
                         │
S101 ◇──────────────────────────────── NO ──→ (1)
         Fdiag = OFF
              │ YES
S102 ◇──────────────────────────────── NO ──────────────┐
         IS SENSOR                                       │
         ELEMENT ACTIVE?                                 │
              │ YES                                      │
S103 ◇──────────────────────────────── NO ──────────────┤
         IS FAILURE                                      │
         DETECTION CONDITION                             │
         ESTABLISHED?                                    │
              │ YES                                      │
S104 ┌────────────────────────────┐                     │
     │ START OPEN-LOOP CONTROL     │                     │
     └────────────────────────────┘                     │
S105 ┌────────────────────────────┐                     │
     │      Vev ← Vpre             │                     │
     └────────────────────────────┘                     │
              │                                          │
              ↓←──────────────────────────────┐         │
S106 ◇──────────────────────────── NO ────────┘         │
         HAS Δt ELAPSED?                                 │
              │ YES                                      │
S107 ┌────────────────────────────┐                     │
     │      ACQUIRE Idet           │                     │
     └────────────────────────────┘                     │
S108 ◇──────────────────────────── NO ──────┐           │
         Idet > Ithre                        │           │
              │ YES                          │           │
S109 ┌─────────────────────┐   S111 ┌─────────────────────┐
     │ DETERMINE THAT       │       │ DETERMINE THAT       │
     │ SENSOR FAILS         │       │ SENSOR IS NORMAL     │
     └─────────────────────┘   S112 └─────────────────────┘
S110 ┌─────────────────────┐       ┌─────────────────────┐
     │ TURN ON WARNING LAMP │       │      Vev ← Vtrg      │
     └─────────────────────┘   S113 └─────────────────────┘
              │                     ┌─────────────────────┐
              │                     │ RESUME F/B CONTROL   │
              │                     └─────────────────────┘
              │←──────────────────────────┘
S114 ┌────────────────────────────┐
     │      Fdiag ← ON             │
     └────────────────────────────┘
              │
 (1) ─────────→│
S115 ◇──────────────────────────────── NO ──────────────┐
         IS ENGINE STOPPED?                              │
              │ YES                                      │
S116 ┌────────────────────────────┐                     │
     │      Fdiag ← OFF            │                     │
     └────────────────────────────┘                     │
              │←─────────────────────────────────────────┘
         ┌──────────┐
         │   END    │
         └──────────┘
```

22

# FIG. 10

# FIG. 11

# FIG. 12

START

S101 — Fdiag = OFF — NO → (1)
↓ YES

S102 — IS SENSOR ELEMENT ACTIVE? — NO
↓ YES

S103 — IS FAILURE DETECTION CONDITION ESTABLISHED? — NO
↓ YES

S104 — START OPEN-LOOP CONTROL

(2) →

S201 — Tsens ≥ Tpre — NO
↓ YES                    S202 — EXECUTE HEATING PROCESSING
S105 — Vev ← Vpre                          ↓
                                          (2)

S106 — HAS Δt ELAPSED? — NO
↓ YES

S107 — ACQUIRE Idet

S203 — Idet > Ithre' — NO
↓ YES

S109 — DETERMINE THAT SENSOR FAILS          S111 — DETERMINE THAT SENSOR IS NORMAL

                                            S112 — Vev ← Vtrg

S110 — TURN ON WARNING LAMP                 S113 — RESUME F/B CONTROL

S114 — Fdiag ← ON

(1) →

S115 — IS ENGINE STOPPED? — NO
↓ YES

S116 — Fdiag ← OFF

END

Europäisches
Patentamt

European
Patent Office

Office européen
des brevets

## EUROPEAN SEARCH REPORT

Application Number

EP 19 20 7122

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 3 020 949 A1 (TOYOTA MOTOR CO LTD [JP]) 18 May 2016 (2016-05-18) * figures 2,7-9,10 * * paragraphs [0001], [3150], [0051], [0056] - [0060], [0061], [0063], [0064], [0073] * * table 1 * | 1-4,6-9 | INV. G01N27/417 |
| X | EP 3 023 620 A1 (TOYOTA MOTOR CO LTD [JP]) 25 May 2016 (2016-05-25) * figures 1,2,6,11C * * paragraphs [0010], [0020], [0034], [0049] * | 1-5,8,9 | |
| X | US 4 886 028 A (UCHINAMI MASANOBU [JP] ET AL) 12 December 1989 (1989-12-12) * figure 6 * * column 1, lines 7-8,60-65 * * claim 1 * | 1,9 | |
| X | JP 2012 068150 A (TOYOTA MOTOR CORP) 5 April 2012 (2012-04-05) * figures 1,2,13 * * paragraphs [0032], [0033], [0093], [0107] * | 1,9 | TECHNICAL FIELDS SEARCHED (IPC) G01N F02D |

The present search report has been drawn up for all claims

| Place of search | Date of completion of the search | Examiner |
|---|---|---|
| Munich | 26 February 2020 | Kratz, Dorothee |

EPO FORM 1503 03.82 (P04C01)

**EP 3 650 848 A1**

## ANNEX TO THE EUROPEAN SEARCH REPORT
## ON EUROPEAN PATENT APPLICATION NO.

EP 19 20 7122

This annex lists the patent family members relating to the patent documents cited in the above-mentioned European search report.
The members are as contained in the European Patent Office EDP file on
The European Patent Office is in no way liable for these particulars which are merely given for the purpose of information.

26-02-2020

| Patent document cited in search report | | Publication date | Patent family member(s) | | Publication date |
|---|---|---|---|---|---|
| EP 3020949 | A1 | 18-05-2016 | CN | 105587419 A | 18-05-2016 |
| | | | EP | 3020949 A1 | 18-05-2016 |
| | | | JP | 6311578 B2 | 18-04-2018 |
| | | | JP | 2016089799 A | 23-05-2016 |
| | | | US | 2016131064 A1 | 12-05-2016 |
| EP 3023620 | A1 | 25-05-2016 | CN | 105545514 A | 04-05-2016 |
| | | | EP | 3023620 A1 | 25-05-2016 |
| | | | JP | 6222037 B2 | 01-11-2017 |
| | | | JP | 2016084725 A | 19-05-2016 |
| | | | US | 2016115893 A1 | 28-04-2016 |
| US 4886028 | A | 12-12-1989 | JP | H01219328 A | 01-09-1989 |
| | | | US | 4886028 A | 12-12-1989 |
| JP 2012068150 | A | 05-04-2012 | NONE | | |

EPO FORM P0459

For more details about this annex : see Official Journal of the European Patent Office, No. 12/82

**REFERENCES CITED IN THE DESCRIPTION**

**Patent documents cited in the description**

- JP 2004019542 A